# FASCICULE DE BREVET EUROPEEN

(11) **EP 0 318 524 B1**
(45) Date de publication et mention de la délivrance du brevet: **27.01.1993**
(21) Numéro de dépôt: 87907227.0
(22) Date de dépôt: 26.10.1987
(51) Int. Cl.: A61F 11/00

(54) **DISPOSITIF POUR SUPPRIMER LES MAUX D'OREILLES, PRODUITS PAR DES VARIATIONS DE PRESSION DE L'ATMOSHPERE AMBIANTE**
VORRICHTUNG ZUR VERMEIDUNG VON OHRENSCHMERZEN, VERURSACHT DURCH ÄNDERUNGEN DES UMGEBUNGSDRUCKS
DEVICE FOR SUPPRESSING EAR TROUBLES PRODUCED BY PRESSURE VARIATIONS OF THE ENVIRONMENTAL ATMOSPHERE

(30) Priorité: 27.10.1986 FR 8614926
(43) Date de publication de la demande: 07.06.1989
(73) Titulaire: ROSSI, Jean-Pierre, F-93190 Livry-Gargan (FR); ROSSI, Marie-Jose, F-93190 Livry-Gargan (FR)
(72) Inventeur: ROSSI, Jean-Pierre, F-93190 Livry-Gargan (FR); ROSSI, Marie-Jose, F-93190 Livry-Gargan (FR)
(74) Mandataire: Portal, Gérard
(86) Numéro de dépôt international: FR8700416
(87) Numéro de publication internationale: WO8803010

(56) Documents cités:
- US-A- 1 805 471

## Description

La présente invention concerne les dispositifs pour supprimer les perturbations du fonctionnement du système auditif de certaines personnes, occasionnées, par exemple,lors de déplacements dans des moyens de transports individuels ou collectifs, comme les aéronefs, en assurant la suppression, par exemple, des souffrances auxquelles elles sont soumises, notamment à cause d'une mauvaise pressurisation du milieu ambiant. En certaines circonstances, de nombreuses personnes souffrent de maux d'oreilles, notamment quand elles se déplacent ou voyagent à haute altitude en avion. Ce mal est généralement dû au manque ou à l'insuffisance de préssurisation. Notamment en avion, il est indispensable, pour le confort et la santé des voyageurs, de maintenir une pression normale et sensiblement constante à l'intérieur de l'habitacle, surtout pendant le laps de temps, plus ou moins long, compris entre le décollage et le moment ou l'avion parvient à son altitude de croisière. Cette opération est actuellement effectuée sur tout le volume de l'appareil, ce qui implique que, parfois, l'on n'obtienne pas une parfaite préssurisation. Ce défaut occasionne des malaises parfois insupportables pouvant même obliger certaines personnes à renoncer à ce mode de transport.

La présente invention a pour but de réaliser un dispositif plus particulièrement destiné aux transports aériens, mais convient très bien à d'autres modes de transports, tels que :voiture, train, bateau (mal des transports), ainsi qu'à la pêche sous-marine, au déplacement en montagne (alpinisme), etc. qui soit d'une conception très simple et qui puisse être très facilement utilisé par toute personne en tout lieu.

Suivant une autre fonction de la même invention, l'appareil, fonctionnant selon le même principe, permet à tout utilisateur de faire abstraction des bruits environnants et ce grace au vide d'air que procure cet appareil.

Pour ce faire, l'invention utilise un dispositif adapté à établir et maintenir dans les conduits auditifs d'une personne, une pression differente de la present ambiante.

US-A-1805471 décrit, certes, un dispositif adapté à produire, par l'intermédiaire d'une pièce creuse coopérant avec des moyens de pompage munis de moyens anti-retour, une pression différente de la pression ambiante dans un conduit auditif d'une personne, mais ce dispositif, compte-tenu de sa destination, est dépourvu de moyens assurant l'isolement hermétique entre le milieu ambiant et le volume défini par sa cavité et le conduit auditif. Il est donc impropre au maintien, dans le conduit auditif, d'une pression différente de la pression ambiante.

Le dispositif selon l'invention se distingue de l'art antérieur tel que représenté par US-A-1 805 741 par le fait qu'il comporte, comme pièces creuses, au moins une paire d'embouts et/ou de ventouses et chaque pièce de la paire est adaptée à être associée à l'un des conduits auditifs de la personne en établissant, grâce à des moyens de fixation maintenant le dispositif en place, une étanchéité aussi totale que possible vis-à-vis du milieu ambiant, grâce à quoi, en produisant ladite différence de pression, et en la maintenant, il est possible de supprimer les conséquences, telles que douleurs, des perturbations du fonctionnement du système auditif qui peuvent résulter des variations de la pression ambiante.

D'autres caractéristiques et avantages de la présente invention apparaîtront au cours de la description suivante donnée en regard des dessins annexés à titre illustratif mais nullement limitatif dans lesquels :
La figure 1 représente, vue en coupe partielle, un premier mode de réalisation d'un dispositif de l'invention.
La figure 2 représente une partie d'un deuxième mode de réalisation d'un dispositif selon l'invention.
La figure 3 représente, vue en coupe, une partie d'un troisième mode de réalisation d'un dispositif selon l'invention.
La figure 4 représente, à titre d'application et sous forme schématique, un dispositif en accord avec les figures 1 à 3 associé à un aéronef.
La figure 5 représente, à titre d'application et sous forme schématique, un dispositif portatif individuel, conforme à l'invention.

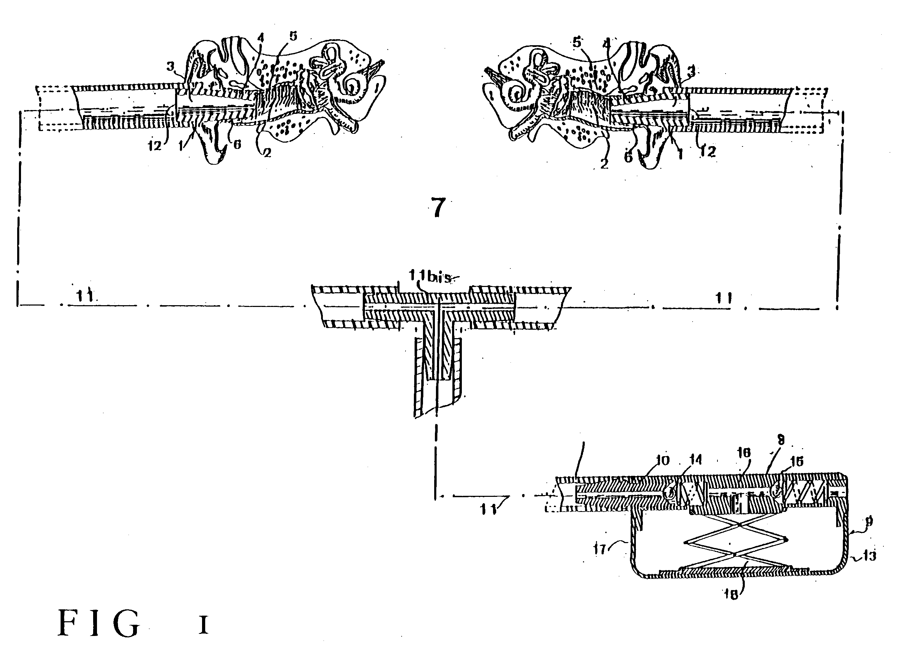

La figure 1 représente un premier mode de réalisation d'un dispositif selon l'invention, qui comporte au moins deux embouts 1 aptes à être associés un à chaque conduit auditif 2 d'une personne, chaque embout comportant un orifice traversant 3. Une des extrémités 4 de chaque embout a avantageusement une forme sensiblement conique, ce qui lui permet d'être facilement enfichée dans le conduit auditif. L'orifice traversant 3 débouche sur la petite base 5 de cette extrémité conique 4. Dans un mode avantageux de réalisation, l'extrêmité conique 4 de l'embout est réalisée en un matériau sensiblement déformable pour pouvoir épouser la surface latérale 6 de l'entrée du conduit auditif 2, afin d'assurer la plus parfaite étanchéité entre l'intérieur de ce conduit 2 et le milieu ambiant 7 dans lequel se trouve la personne utilisant le dispositif. Le dispositif comporte en outre des moyens 8 pour commander une circulation de fluide dans l'orifice traversant 3. Dans le mode de réalisation illustré sur la figure 1, les moyens pour commander une circulation de fluide dans l'orifice traversant comportent au moins une pompe 9 avec une entrée d'aspiration 10 et des moyens de conduit 11, 11 bis, partiellement représentés, pour relier l'entrée 10 de la pompe 9 avec la sortie 12 de l'orifice traversant 3 opposée à celle débouchant sur la petite base 5 de l'embout. Dans le mode illustré, la pompe est une pompe manuelle essentiellement constituée d'une chambre à volume élastiquement déformable 13, et de deux clapets d'aspiration 14 et de refoulement 15, en l'occurence le clapet d'aspiration 14 constituant l'évacuation de l'air, se trouvant dans les moyens de conduit et le conduit auditif, dans la pompe 9 et le clapet de refoulement I5 constituant la sortie de l'air de la pompe 9 vers l'extérieur. Les deux clapets sont réalisés dans une embase rigide 16 sur laquelle est située la chambre 13 qui est, par exemple, constituée d'une cloche 17 en matériau présentant une certaine souplesse sur laquelle s'exerce la force de rappel d'un ressort 18 qui tend à lui donner son volume maximum.

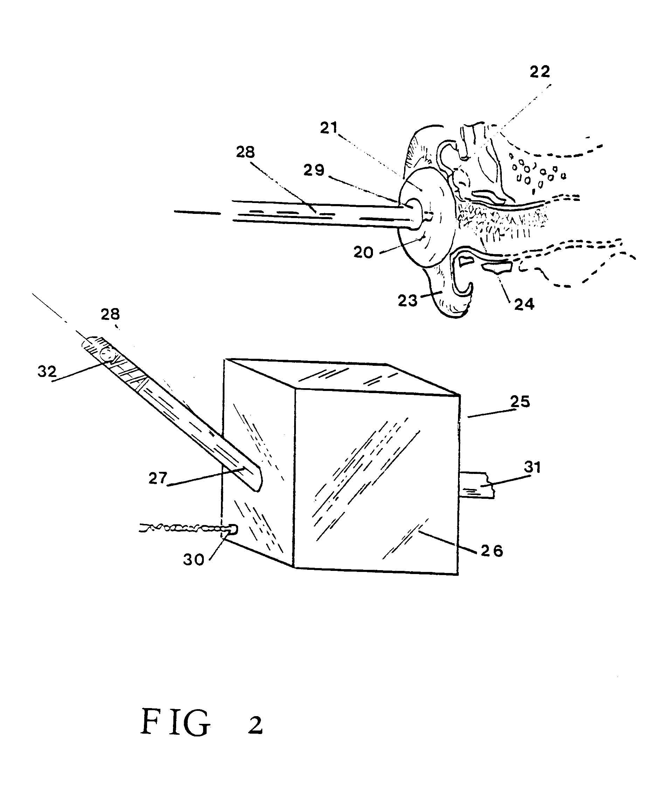

Dans le mode de réalisation selon la figure 1, le dispositif fonctionne de la façon suivante: Lorsqu'un passager emprunte un véhicule tel qu'un aéronef, pour éviter d'avoir des troubles au niveau du conduit auditif, en particulier au niveau du tympan, au moment de l'envol ou de l'atterrissage, il peut utiliser le dispositif tel que décrit et illustré sur la figure 1. Préalablement à l'envol ou à l'atterrissage, il place les embouts 1 respectivement un dans chacun de ses conduits auditifs en enfichant chacune des extrémités coniques 4 dans l'orifice d'entrée du conduit, jusqu'à ce que la paroi de cette extrêmité épouse le plus parfaitement possible la surface latérale 6 du conduit auditif pour assurer, comme dit précédemment, la plus parfaite étanchéité possible, entre l'intérieur du conduit auditif et l'atmosphère ambiante 7 dans laquelle se trouve le passager, en l'occurrence de l'air de l'habitacle de l'aéronef. Lorsque l'aéronef commence à prendre son envol ou à descendre en altitude, et que le passager commence à ressentir des premières douleurs au niveau des tympans, il peut agir sur la chambre à volume variable 13 en déformant la cloche 17 pour, dans un premier temps, comprimer le ressort 18 et donc expulser l'air se trouvant dans cette chambre 13 par le clapet 15. Dans un second temps, en se détendant, le ressort 18 redonne à la chambre 13 son volume initial, ce qui permet de réaliser une aspiration dans le conduit 11 et le té 11 bis et donc, par l'intermédiaire de l'orifice traversant 3, à l'intérieur des conduits auditifs 2. De cette façon, les pressions de part et d'autre du tympan peuvent s'équilibrer et le passager ne ressent plus de douleur. Il pourra bien entendu agir plusieurs fois sur le volume variable de la même façon que décrit ci-dessus, s'il en ressent le besoin. La figure 2 représente un autre mode de réalisation d'un dispositif conforme à l'invention. Dans ce mode de réalisation, les pièces creuses 20 sont constitués de ventouses 21 aptes à être appliquées de façon la plus étanche possible sur la partie 22 de la surface latérale de l'oreille 23 entourant l'ouverture du conduit auditif 24, ou l'oreille entière, l'orifice de circulation du fluide défini ci-avant débouchant sensiblement au centre de la ventouse. Bien entendu, le dispositif comporte aussi ces moyens 25 pour commander cette circulation de fluide dans l'orifice traversant de la ventouse 21. Les moyens 25 pour commander la circulation de fluide dans l'orifice traversant comportent au moins une pompe 26 avec une entrée d'aspiration d'air 27 et une sortie de refoulement d'air 31 et des moyens de conduit 28, incluant un clapet anti retour 32, qui reliant l'entrée 27 de la pompe 26 à la sortie 29 l'orifice traversant, la pompe illustrée étant par exemple une pompe à commande électrique par son éntrée de commande 30. Cependant, s'il n'y a pas la possibilité de l'alimenter en énergie électrique, y compris les piles électriques ou autres sources d'énergie, la pompe électrique peut-être remplacée par une pompe manuelle comme celle décrits en regard de la figure 1.

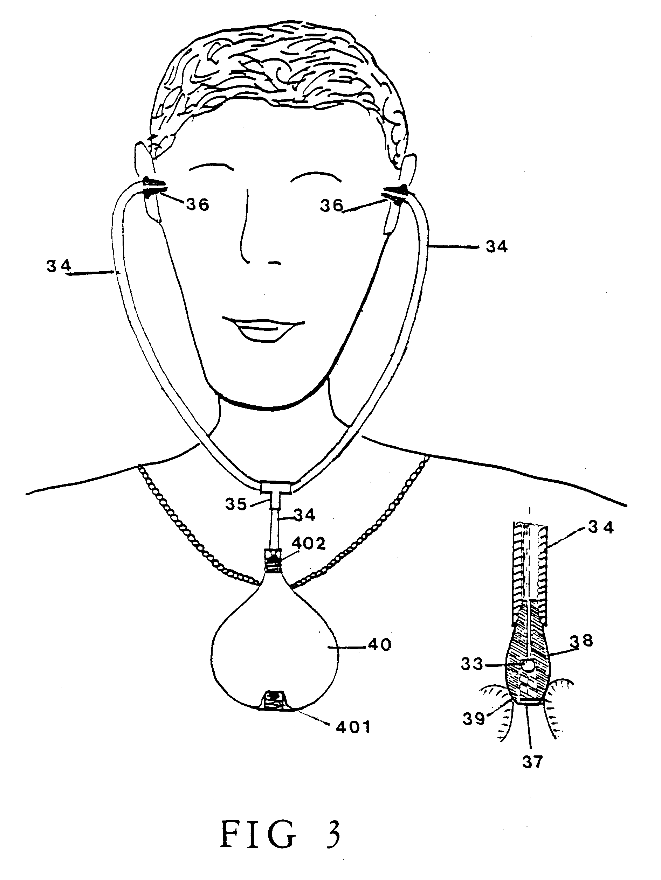

Pour des dispositifs très légers pouvant être emmener en tous lieux, il peut être avantageux de simplifier au maximum la réalisation de la pompe en lui donnant, par exemple, une structure comme celle qui est illustrée sur la figure 3.

Dans ce mode de réalisation, le dispositif est constitué d'un clapet d'aspiration 33 situé a une extrémité de moyens de conduit 34 dont les deux autres extrémités sont reliées chacun à un orifice traversant de l'embout 36 de chaque oreille, par l'intermédiaire d'un té 35. Le clapet est réalisé dans un bec 37 dont les formes extérieures 38 peuvent-être connectées à des moyens d'aspiration par exemple la bouche 39 d'une personne, ou une pompe, par exemple, en forme de poire 40 en caoutchouc ou plastique dotée d'un clapet anti-retour sur l'évacuation d'air 401, et d'un clapet anti-retour sur l aspiration d'air 402, avec ou sans ressort à l'intérieur pour radonner à la chambre son volume initial, de cette façon,la personne elle-même peut doser la pression dans le conduit auditif de son oreille et combattre les douleurs ressenties lors du décollage ou de l'atterrissage d'un aéronef ou dans toutes autres circonstances ou les troubles ou nuisances provenant des oreilles peuvent causées des douleurs,en aspirant directement avec sa bouche dans le bec 37 ou en pressant entre ses mains la poire, et ce jusqu'à ce que les douleurs disparaissent.

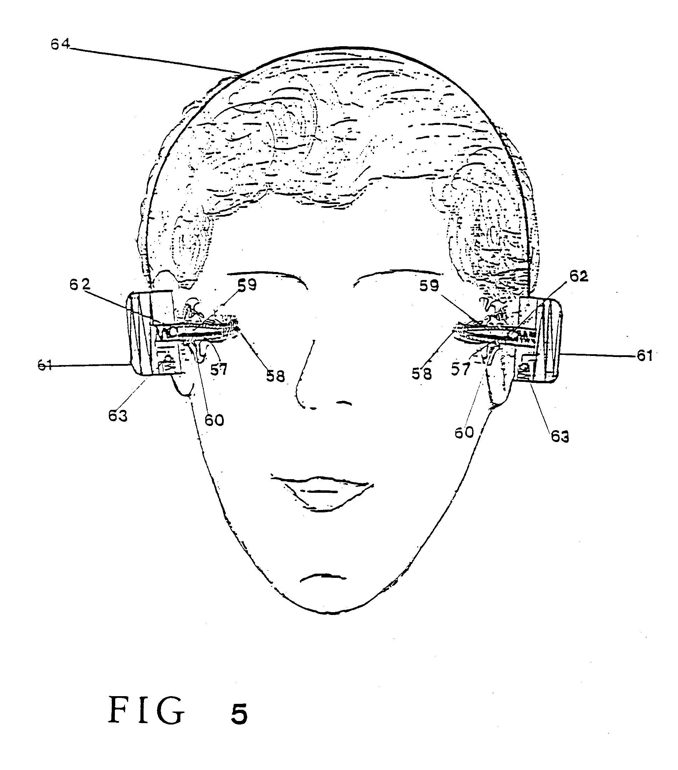

La figure 5 représente un mode de réalisation d'un dispositif selon l'invention, qui comporte deux embouts 57 aptes à être associés, un à chaque conduit auditif 58 d'une personne, chaque embout comportant un orifice traversant 59. Chaque extrémité des embouts à avantageusement une forme sensiblement conique, en un matériau sensiblement déformable, ce qui lui permet d'être facilement enfichée dans le conduit auditif et de pouvoir épouser la surface latérale de l'entrée du conduit auditif 60 en assurant une presque parfaite étanchéité entre l'intérieur de ce conduit et le milieu ambiant dans lequel se trouve la personne utilisant le dispositif. Le dispositif comporte en outre des moyens 61 pour commander une circulation de fluide dans les orifices traversant 59.

Dans le mode de réalisation illustré sur la figure 5, les moyens pour commander une circulation de fluide dans l'orifice traversant comportent au moins deux pompes indépendantes avec une aspiration d'air, munie d'un clapet anti-retour d'air 62, et une sortie d'air, munie d'un clapet anti-retour 63, et des moyens de fixation de l'ensemble de l'appareil 64, en l'occurrence, pour l'exemple, un arceau posé sur la tête qui retient les deux pompes et en même temps les serre relativement fort contre les deux oreilles pour maintenir l'étanchéité.

Dans le mode de réalisation selon la figure 5 les caractéristiques des pompes sont tout à fait identiques à celles de la pompe présentée sur la figure 1, à la différence que les clapets d'aspiration d'air et de refoulement d'air sont réalisés dans une embase, relativement rigide, mais constituée d'un matériau présentant une certaine souplesse pour ne pas blesser les oreilles et assurer le meilleur confort à l'utilisateur, et que le clapet d'aspiration d'air se trouve en plein centre de la pompe dans l'embase rigide et directement relié à l'orifice traversant de l'embout.

Dans le mode de réalisation selon la figure 5, le dispositif fonctionne de la même façon que la pompe de la figure 1, à la différence qu'il faut à l'utilisateur agir simultanément sur les deux pompes à la fois en les pressant contre ses oreilles, pour soulager simultanément ses deux tympans. Il pourra bien entendu agir plusieurs fois sur le volume variable, de la même façon que décrit plus haut, s'il en ressent le besoin. Selon une variante de la figure 5 les pièces creuses peuvent être comme sur la figure 2 constitués de ventouses aptes à être appliquées de façon la plus étanche possible sur la partie de la surface latérale de l'oreille entourant l'ouverture du conduit auditif, ou englobant la totalité de l'oreille, l'orifice de circulation du fluide, défini comme sur la figure 2, débouchant sensiblement au centre de la ventouse. Bien entendu,le dispositif comporte aussi des moyens pour commander cette circulation de fluide dans l'orifice traversant de la ventouse.Ces moyens sont les mêmes que ceux décrits, plus haut, dans la figure 5.

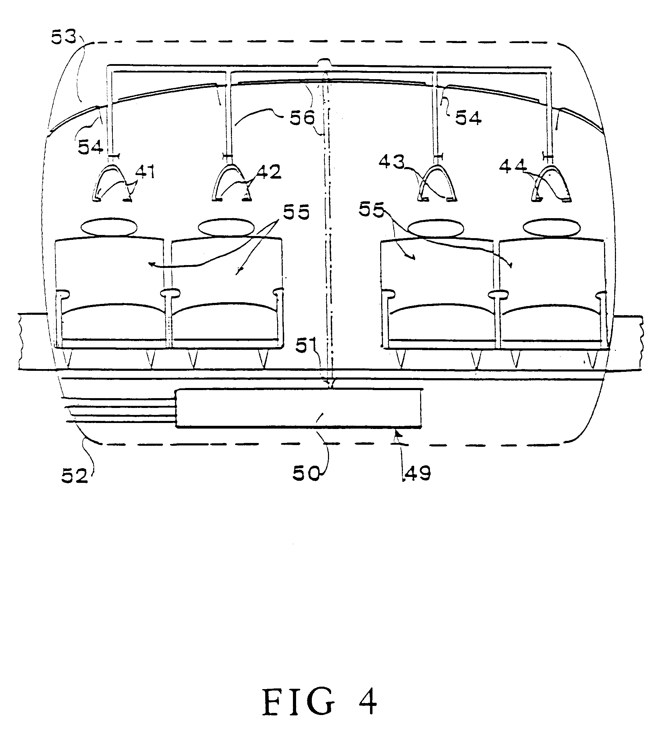

Le dispositif peut même comporter une pluralité d'embouts 41-44, comme représenté sur la figure 4, et des moyens 49 pour commander une circulation de fluide comprenant une pompe 50 reliée par son entrée d'aspiration d'air 51 et des moyens 56 aux orifices traversants réalisés dans les embouts 41-44, embouts éventuellement jetables et interchangeables par mesure d'hygiène. Un tel dispositif trouve inconstestablement une application très avantageuse dans un aéronef 52, schématiquement représenté sur cette figure 4. Dans ce cas, les différents embouts sont, par exemple, situés, à proximité de chaque siège 55 de passagers, dans l'habillage 53 de l'aéronef et accessibles en ouvrant des trappes 54, soit au moment du décollage, soit au moment de l'atterrissage. Dans ce mode de réalisation, la pompe est associée au système de préssurisation de l'aéronef. Dans un autre mode de réalisation la pompe pourrait très bien être un groupe à part et totalement indépendant du circuit de préssurisation de l'aéronef pour le cas ou ce dernier tomberait, dans l'hypothèse , en panne. Ce groupe, par exemple, pourrait avoir aussi plusieurs entrées d'aspiration d'air.

## Revendications

1. Dispositif adapté à produire, par l'intermédiaire d'une pièce creuse coopérant avec des moyens de pompage munis de moyens anti-retour (14,15; 31,32; 33; 62,63; 40̸1,40̸2) , une pression différente de la pression ambiante dans un conduit auditif d'une personne, caractérisé en ce qu'il comporte, comme pièces creuses (1,21,36,41-44,57), au moins une paire d'embouts et/ou de ventouses et chaque pièce de la paire est adaptée à être associée à l'un des conduits auditifs (2,24,58) de la personne en établissant, grâce à des moyens de fixation (4-64) maintenant le dispositif en place, une étanchéité aussi totale que possible vis-à-vis du milieu ambiant, grâce à quoi, en produisant ladite différence de pression, et en la maintenant, il est possible de supprimer les conséquences, telles que douleurs, des perturbations du fonctionnement du système auditif qui peuvent résulter des variations de la pression ambiante.

2. Dispositif selon la revendication 1, caractérisé en ce que lesdites pièces creuses sont constituées d'au moins une paire d'embouts (1,36,41-44,57) à extrémité sensiblement conique (4) aptes à être chacun enfiché dans l'un desdits conduits auditifs (2,58) et en ce que ladite extrémité conique (4) est réalisée en un matériau sensiblement déformable pour pouvoir épouser la surface latérale (6) dudit conduit auditif (2, 58) et former moyen de fixation du dispositif en place, assurant ainsi la plus parfaite étanchéité et le meilleur confort.

3. Dispositif selon la revendication 1, caractérisé en ce que lesdites pièces creuses sont constituées d'au moins une paire d'embouts (1,36,41-44,57) aptes à être chacun enfiché dans l'un desdits conduits auditifs (2,58), lesdits moyens assurant l'étanchéité et le maintien du dispositif en place étant constitués par un arceau (64) aux extrémités duquel lesdits embouts sont fixés.

4. Dispositif selon la revendication 1, caractérisé en ce que lesdites pièces creuses sont constituées d'au moins une paire de ventouses (21) aptes à être appliquées, chacune, de façon aussi étanche que possible sur la partie (22) de la surface latérale de l'oreille (23) entourant l'entrée dudit conduit auditif (24), ou englobant l'oreille entière, lesdits moyens assurant l'étanchéité et le maintien du dispositif en place étant constitués par un arceau (64) aux extrémités duquel lesdites ventouses sont fixées.

5. Dispositif selon la revendication 4, caractérisé en ce que chaque ventouse entoure un embout (57) adapté à être enfiché dans le conduit auditif de l'oreille équipée de ladite ventouse.

6. Dispositif selon l'une quelconque des revendications 1 à 5, caractérisé en ce que lesdits moyens de pompage (9,26,40̸,61) sont communs à au moins une paire desdites pièces creuses.

7. Dispositif selon l'une quelconque des revendications 1 à 5, caractérisé en ce que chacune desdites pièces creuses (57) d'une paire est munie de ses propres moyens de pompage (61) , grâce à quoi la variation de pression établie et maintenue peut être différente d'un conduit auditif à l'autre.

8. Dispositif selon la revendication 7, caractérisé en ce que chacune desdites pièces creuses (57) et les moyens de pompage (61) qui lui sont propres sont inclus dans un même boîtier.

9. Dispositif selon l'une quelconque des revendications 1 à 8, caractérisé en ce que lesdits moyens de pompage sont constitués d'une pompe manuelle (9,40̸,61).

10. Dispositif selon l'une quelconque des revendications 1 à 8, caractérise en ce que lesdits moyens de pompage sont constitués par une pompe à fonctionnement électrique (26).

11. Utilisation du dispositif selon l'une quelconque des revendications 1 à 6 pour la mise à disposition des passagers d'un aéronef pressurisé, comprenant les étapes suivantes :
associer lesdits moyens de pompage comportant une pompe au système de pressurisation dudit aéronef
mettre une paire de pièces creuses (41-44) à proximité de chaque siège (55) de passager.

12. Dispositif selon l'une quelconque des revendications 1 à 6, caractérisé en ce que lesdits moyens de pompage comprennent un bec (37) adapté à coopérer avec une poire manuelle (40̸) ou à être pris entre les lèvres (39) de l'utilisateur pour une aspiration à la bouche.

13. Dispositif selon l'une quelconque des revendications 1 à 8, caractérisé en ce que lesdits moyens de pompage comportent une pompe étalonnée et réglée en fonction de son utilisation.

14. Dispositif selon l'une quelconque des revendications 1 à 13, caractérisé en ce que lesdites pièces creuses (1,36,41-44,57) sont constitués de pièces remplaçables et jetables.

## Claims

1. Device adapted to produce, using a hollow part interacting with pumping means fitted with nonreturn means (14, 15; 31, 32; 33; 62, 63; 401, 402), a pressure different from the ambient pressure in an auditory meatus of a person, characterised in that it comprises, as hollow parts (1, 21, 36, 41-44, 57), at least one pair of tips and/or suckers and each part of the pair is adapted to be associated with one of the auditory meatuses (2, 24, 58) of the person while establishing, by virtue of the fastening means (4-64) holding the device in place, as complete leaktightness as possible with respect to the environment around the person, by virtue of which, by producing the said pressure difference, and holding it, it is possible to suppress the consequences, such as pain, of the disturbances in the functioning of the auditory system which may result from variations in the ambient pressure.

2. Device according to Claim 1, characterised in that the said hollow parts are constituted by at least one pair of tips (1, 36, 41-44, 57) with substantially conical ends (4) each capable of being plugged into one of the said auditory meatuses (2, 58) and in that the said conical end (4) is made of a material which is substantially deformable in order to be able to match the lateral surface (6) of the said auditory meatus (2, 58) and to form a means for fastening the device in place, thus providing the most complete leaktightness and the greatest comfort.

3. Device according to Claim 1, characterised in that the said hollow parts are constituted by least one pair of tips (1, 36, 41-44, 57) each capable of being plugged into one of the said auditory meatuses (2, 58), the said means providing the leaktightness and the holding of the device in place being constituted by an open hoop (64) to the ends of which the said tips are fixed.

4. Device according to Claim 1, characterised in that the said hollow parts are constituted by at least one pair of suckers (21) each capable of being applied, in as leaktight a manner as possible onto the portion (22) of the lateral surface of the ear (23) surrounding the entrance of the said auditory meatus (24), or taking in the whole ear, the said means providing the leaktightness and the holding of the device in place being constituted by an open hoop (64) to the ends of which the said suckers are fixed.

5. Device according to Claim 4, characterised in that each sucker surrounds a tip (57) adapted to be plugged into the auditory meatus of the ear fitted with the said sucker.

6. Device according to any one of Claims 1 to 5, characterised in that the said pumping means (9, 26, 40, 61) are common to at least one pair of the said hollow parts.

7. Device according to any one of Claims 1 to 5, characterised in that each of the said hollow parts (57) of one pair is fitted with its own pumping means (61), by virtue of which the pressure variation established and held may be different from one auditory meatus to the other.

8. Device according to Claim 7, characterised in that each of the said hollow parts (57) and the pumping means (61) which belong to it are included in the same case.

9. Device according to any one of Claims 1 to 8, characterised in that the said pumping means are constituted by a manual pump (9, 40, 61).

10. Device according to any one of Claims 1 to 8, characterised in that the said pumping means are constituted by an electrically operated pump (26).

11. Use of the device according to any one of Claims 1 to 6 for making it available to the passengers of a pressurised aircraft, comprising the following steps:
associating the said pumping means comprising a pump with the pressurisation system of the said aircraft
putting a pair of hollow parts (41-44) in proximity to each passenger seat (55).

12. Device according to any one of Claims 1 to 6, characterised in that the said pumping means comprise a spout (37) adapted to interact with a manual pear-shaped pump (14) or to be taken between the lips (39) of the user for oral suction.

13. Device according to any one of Claims 1 to 8, characterised in that the said pumping means comprise a pump which is calibrated and regulated as a function of its use.

14. Device according to any one of Claims 1 to 13, characterised in that the said hollow parts (1, 36, 41-44, 57) consist of replaceable and disposable parts.

## Patentansprüche

1. Vorrichtung zur Erzeugung eines vom Umgebungsdruck abweichenden Drucks im Gehörgang einer Person über ein Hohlteil, das mit mit Rückschlageinrichtungen (14, 15; 31, 32; 33; 62, 63; 401, 402) versehenen Pumpeinrichtungen zusammenwirkt, dadurch gekennzeichnet, daß sie als Hohlteile (1, 21, 36, 41-44, 57) wenigstens ein Paar von Aufsätzen und/oder Saugnäpfen umfaßt und jedes Teil des Paars mit einem der Gehörgänge (2, 24, 58) der Person verbunden werden kann, indem mit Hilfe von Befestigungsmitteln (4-64), die die Vorrichtung an ihrem Platz halten, eine möglichst vollständige Abdichtung gegenüber dem umgebenden Medium hergestellt wird, so daß es durch Erzeugung und Aufrechterhaltung der Druckdifferenz möglich ist, die Folgen von Arbeitsstörungen des Gehörsystems wie Schmerzen zu vermeiden, die durch Änderungen des Umgebungsdrucks entstehen können.

2. Vorrichtung nach Anspruch 1, dadurch gekennzeichnet, daß die Hohlteile wenigstens aus einem Paar von Aufsätzen (1, 36, 41-44, 57) mit im wesentlichen konischem Ende (4) bestehen, die jeweils in einem der Gehörgänge (2, 58) eingesteckt werden können, und daß das konische Ende (4) aus einem im wesentlichen verformbaren Werkstoff besteht, um sich an die Seitenfläche (6) des Gehörgangs (2, 58) anschmiegen und ein Mittel zur Befestigung der Vorrichtung an ihrem Platz formen zu können, so daß es die vollständigste Abdichtung und den besten Komfort gewährleisten kann.

3. Vorrichtung nach Anspruch 1, dadurch gekennzeichnet, daß die Hohlteile aus wenigstens einem Paar von Aufsätzen (1, 36, 41-44, 57) bestehen, die jeweils in einen der Gehörgänge (2, 58) eingesteckt werden können, wobei die Mittel zur Abdichtung und zum Halt der Vorrichtung an ihrem Platz aus einem Bügel (64) bestehen, an dessen Enden die Aufsätze befestigt sind.

4. Vorrichtung nach Anspruch 1, dadurch gekennzeichnet, daß die Hohlteile aus wenigstens einem Paar von Saugnäpfen (21) bestehen, die jeweils so dicht wie möglich auf den den Eingang des Gehörgangs (24) umgebenden Teil (22) der Seitenfläche des Ohrs (23) oder auf das ganze Ohr aufgesetzt werden können, wobei die Mittel zur Abdichtung und zum Halt der Vorrichtung an ihrem Platz aus einem Bügel (64) bestehen, an dessen Enden die Saugnäpfe befestigt sind.

5. Vorrichtung nach Anspruch 4, dadurch gekennzeichnet, daß jeder Saugnapf einen Aufsatz (57) umgibt, der in dem Gehörgang des mit dem Saugnapf ausgerüsteten Ohrs eingesteckt werden kann.

6. Vorrichtung nach einem der Ansprüche 1 bis 5, dadurch gekennzeichnet, daß die Pumpeinrichtungen (9, 26, 40, 61) wenigstens einem Paar von Hohlteilen gemeinsam sind.

7. Vorrichtung nach einem der Ansprüche 1 bis 5, dadurch gekennzeichnet, daß jedes der Hohlteile (57) eines Paars mit seinen eigenen Pumpeinrichtungen (61) ausgerüstet ist, wodurch die erzeugte und aufrechterhaltene Druckänderung von einem Gehörgang zum anderen verschieden sein kann.

8. Vorrichtung nach Anspruch 7, dadurch gekennzeichnet, daß jedes der Hohlteile (57) und die ihm eigenen Pumpeinrichtungen (61) in ein gemeinsames Gehäuse eingeschlossen sind.

9. Vorrichtung nach einem der Ansprüche 1 bis 8, dadurch gekennzeichnet, daß die Pumpeinrichtungen aus einer manuellen Pumpe (9, 40, 61) bestehen.

10. Vorrichtung nach einem der Ansprüche 1 bis 8, dadurch gekennzeichnet, daß die Pumpeinrichtungen aus einer elektrisch arbeitenden Pumpe (26) bestehen.

11. Verwendung der Vorrichtung nach einem der Ansprüche 1 bis 6 für die Bereitstellung für Passagiere eines Luftfahrzeugs mit Druckanlage, bestehend aus den folgenden Schritten:
die eine Pumpe umfassenden Pumpeinrichtungen mit dem Drucksystem des Luftfahrzeugs verbinden,
ein Paar von Hohlteilen (41-44) in Nähe jedes Passagiersitzes (55) anordnen.

12. Vorrichtung nach einem der Ansprüche 1 bis 6, dadurch gekennzeichnet, daß die Pumpeinrichtungen ein Mundstück (37) umfassen, das mit einem handbetätigten Ball (40) zusammenwirken oder für eine Absaugung mit dem Mund zwischen die Lippen (39) des Benutzers genommen werden kann.

13. Vorrichtung nach einem der Ansprüche 1 bis 8, dadurch gekennzeichnet, daß die Pumpeinrichtungen eine geeichte und in Abhängigkeit von ihrer Verwendung eingestellte Pumpe umfassen.

14. Vorrichtung nach einem der Ansprüche 1 bis 13, dadurch gekennzeichnet, daß die Hohlteile (1, 36, 41-44, 57) aus austauschbaren Wegwerfteilen bestehen.
